# EUROPEAN PATENT APPLICATION

(11) **EP 1 374 879 A1**
(43) Date of publication of application: **02.01.2004**
(21) Application number: 02703914.8
(22) Date of filing: 05.03.2002
(51) Int. Cl.: A61K 35/74, A61K 31/711, A61P 31/04, A61P 1/00

(54) **BACTERIAL TRANSLOCATION INHIBITOR AND METHOD OF INHIBITING BACTERIAL TRANSLOCATION**

(30) Priority: 05.03.2001 JP 2001109213
(71) Applicant: Calpis Co., Ltd., Shibuya-ku, Tokyo 150-0021 (JP)
(72) Inventor: MARUBASHI, Toshihiro, Sagamihara-shi, Kanagawa 228-0812 (JP); IMABAYASHI, Tomokazu, Yamato-shi, Kanagawa 242-0001 (JP); MARUTA, Kiyoshi, Machida-shi, Tokyo 194-0045 (JP)
(74) Representative: Hallybone, Huw George
(86) International application number: PCT/JP2002/002005
(87) International publication number: WO 2002/069991

(57) **Abstract**

A bacterial translocation inhibitor containing live Bacillus subtilis cells as an active ingredient, and a method for inhibiting bacterial translocation comprising the step of orally administering the bacterial translocation inhibitor.

## Description

The present invention relates to a bacterial translocation inhibitor for inhibiting bacterial invasion via intestinal barrier, and a method for inhibiting bacterial translocation.

Most of the microorganisms that come into contact with animals including humans are those present in the intestinal tract of the animals. With humans, for example, these microorganisms number up to 10⁵-10⁷ in the lower portion of the small intestine and 10⁹-10¹¹ in the large intestine per 1 g of intestinal tract contents. Microbial strains are also said to number roughly 100 strains. These microorganisms include harmful bacteria that invade other organisms and then multiply, initiating what is known as opportunistic infection or serious illnesses such as multiple organ failure and septicemia.

One method of inhibiting these illnesses is thought to be inhibition of the penetration through intestinal mucosa and invasion of the abdominal cavity and organs by these harmful bacteria.

Mechanisms of achieving inhibition of such invasion are called the intestinal barrier. The intestinal barrier includes an intestinal immune system and anatomical structures.

The intestinal immune system is an immune system specific to the vicinity of the intestinal tract involving gut-associated lymphoid tissue, secretory IgA, and phagocytic cells. The anatomical structures of the intestinal barrier are structures which physically inhibit the penetration through intestinal mucosa by bacteria, such as mucous layers and epithelial cells.

Specifically, examples of these structures are carbohydrate complexes containing proteins, glycolipids, and glycosaminoglycans that are present in the cell surface, forming part of the cell membrane. These complexes are called the glycocalyx. The development of the glycocalyx physically prevents contact and attacks by bacteria and improves the intestinal barrier function of mucosal cells, thus decreasing the frequency of necrosis and desquamation of epithelial cells and decreasing the burden on the host organism.

Bacterial invasion of the host organism via the intestine barrier is called bacterial translocation (BTL). There are two possible BTL routes, which are lymphatic system and blood vessel. The lymphatic system is considered to be the main route based on many animal tests and clinical researches. It appears that the bacteria enter in lymphatic route in the case of BTL caused by aberrant growth of bacteria with minor mucous damage, whereas the bacteria also directly enter in blood flow in the case of BTL with severe invasion such as hemorrhagic shocks. Lymphatic BTL in which bacterial invasion is limited to mesenteric lymph nodes (abbreviated hereinbelow as MLN) themselves does not result in serious condition. However, when the amount of the bacteria in MLN exceeds treatable amount, the bacteria further invade vascular system and organs, which results in serious diseases. Therefore, inhibition of BTL into MLN leads to prevention of diseases caused by BTL.

BTL in animals raised for meat results in not only contamination of edible portion which may cause food poisoning, but also inhibition of optimal growth of the animals which may cause low productivity.

Methods for treating humans or animals with BTL in the prior art may include administration of antibiotics, vaccines, and interferon. However, administration of antibiotics may cause problems of occurrence of antibiotics resistant bacterial strains and side effects. Since the effect of a vaccine is based on the specific action against each contagium, vaccines for all contagiummust be administered for inhibition of BTL by vaccines . Since interferon is expensive, use thereof is limited to human and not applicable to animals raised for meat in terms of cost.

The object of the present invention is to provide a medicament which can inhibit BTL easily and effectively, as well as an easy and effective method for inhibiting BTL.

According to the present invention, there is provided a BTL inhibitor containing live Bacillus subtilis cells as an active ingredient.

According to the present invention, there is also provided the BTL inhibitor wherein the Bacillus subtilis is Bacillus subtilis C-3102 (deposited at National Institute of Bioscience and Human-Technology with deposition number FERM BP-1096).

According to the present invention, there is further provided the BTL inhibitor wherein said Bacillus *subtilis* C-3102 has a chromosomal DNA which gives a DNA fragment of about 700 bps when said chromosomal DNA is extracted, purified and subjected to PCR treatment using primers of Sequences 1 and 2.

According to the present invention, there is further provided a method for inhibiting BTL comprising the step of orally administering the BTL inhibitor.
Fig. 1 is a microscopic photograph showing section of ileum of healthy control group in Example 3.
Fig. 2 is an illustration of necrosis, ablation and desquamation of mucous epithelial cells and infiltration of neutrophiles and lymphocytes into lamina propria observed in Fig.1.
Fig. 3 is a microscopic photograph showing section of ileum of healthy intake group in Example 3.
Fig. 4 is an illustration of necrosis, ablation and desquamation of mucous epithelial cells and infiltration of neutrophiles and lymphocytes into lamina propria observed in Fig.3.
Fig. 5 is a microscopic photograph showing section of caecum of healthy control group in Example 3.
Fig. 6 is an illustration of necrosis, ablation and desquamation of mucous epithelial cells and infiltration of neutrophiles and lymphocytes into lamina propria observed in Fig. 5.
Fig. 7 is a microscopic photograph showing section of caecum of healthy intake group in Example 3.
Fig. 8 is an illustration of necrosis, ablation and desquamation of mucous epithelial cells and infiltration of neutrophiles and lymphocytes into lamina propria observed in Fig.7.
Fig. 9 is a photograph showing results of electrophoresis of products obtained by PCR amplification in Experiment 1.

The BTL inhibitor of the present invention contains live *Bacillus subtilis* cells as an active ingredient. As used herein, "live *Bacillus subtilis* cells" means live spores and/or vegetative cells of *Bacillus subtilis.*

Bacteriological characteristics of *Bacillus subtilis* is described in, e.g., Bergey's Manual of Bacteriology Vol.11 (1986). As the live bacteria, those having the following characteristics may be used:
(1) Gram positive
(2) Forming oval shape spore
(3) Rod shape bacteria
(4) Mobility: positive
(5) Aerobic
(6) Catalase: positive
(7) Growth at 50°C: +
(8) Growth at pH 5.7: +
(9) Utilization of citrate: +
(10) Generation of acid from : arabinose, glucose, xylose and mannitol: +
(11) VP test: +
(12) Hydrolysis of starch: +
(13) Reduction of nitrate: +
(14) Formation of indole: -
(15) Hydrolysis of gelatin: +
(16) Hydrolysis of casein: +
(17) Formation of membrane in liquid medium: +
(18) Coagulation of milk: -
(19) Peptonization of milk: +

BTL inhibiting effect of *Bacillus subtilis* varies to a certain extent depending on the type of strain. The preferable. strain of *Bacillus subtilis* which gives the active ingredient of the present BTL inhibitor may include *Bacillus subtilis* C-3102. *Bacillus subtilis* C-3102 was deposited at National Institute of Bioscience and Human-Technology of Agency of the Industrial Science and Technology with deposition number FERM BP-1096 on December 25, 1985. National Institute of Bioscience and Human-Technology has changed its name to International Patent Organism Depositary of National Institute of Advanced Industrial Science and Technology (AIST Tsukuba Central 6, 1-1, Higashi 1 chome, Tsukuba-shi, Ibaraki-ken, Japan). *Bacillus subtilis* C-3102 is now publicly available from International Patent Organism Depositary.

*Bacillus subtilis* C-3102 is characterized by amplification of fragments of about 700 bps when a PCR reaction of its chromosome DNA is performed using PCR primers with Sequences 1 and 2 as listed below. Primers having Sequences 1 and 2 were originally developed as primers amplifying DNA coding for *Bacillus amyloliquefaciens∼amylase.* With other Bacillus subtilis strains, amplification with theses PCR primers does not occur. Fragments of about 700 bps grown in Bacillus subtilis C-3102 are characterized by a lack of homology with the amylase sequence and are clearly distinguished from other Bacillus subtilis strains.
Sequence 1: 5'-GCCCCGCACATACGAAAAGACTGGCTGAAA-3'
Sequence 2: 5'-GGATCCCACGTTGTGATTAAAAGCAGCGAT-3'

The aforementioned *Bacillus subtilis* may be cultured using a liquid or solid medium containing a carbon source, a nitrogen source, and inorganic substances normally used as a culture medium in microbial culturing. The carbon source may be those *Bacillus subtilis* can utilize, such as glucose, fructose, sucrose, starch, and molasses. Examples of the nitrogen source may include peptone, casein hydrolysate, meat extract, and ammonium sulfate. The medium may further contain salts of phosphoric acid, potassium, magnesium, calcium, sodium, iron, and manganese, vitamins, amino acids, and detergents. Aerobic conditions are preferable as culturing conditions. Examples of the preferable culture apparatus may include a liquid culture apparatus with aerated agitation such as a jar fermenter, a shelf-modelsolid culture apparatus, and a koji-making apparatus . A culturing temperature of 20-50°C and particularly 30-45°C is preferable. The culturing time may be from 12 hours to 7 days. The initial pH for culturing may be pH 5-9, and pH 6-8 is particularly preferable.

Cultures obtained via the aforementioned methods may be used themselves as the BTL inhibitor of the present invention. Concentrates of the cultures, cell bodies isolated from the cultures, and formulations such as dried powders, granules, and tablets further containing additives such as excipient may also be used as the BTL inhibitor of the present invention. The excipient may include calcium carbonate, corn grits, cornflour, defatted rice bran, wheat bran and dried skim milk, but not limited thereto.

The BTL inhibitor of the present invention preferably contains 10⁶-10¹¹ cells/g of live *Bacillus subtilis* cells, i.e. spores and/or vegetative cells.

Subjects to which the BTL inhibitor of the present invention is administered may include animals such as those raised for meat such as cows, pigs and chickens, as well as humans, but not limited thereto. Of these subjects for administration, those with a high risk of BTL are particularly preferred, such as subjects having an abnormal proliferation of intestinal bacteria, decreased host immune functions, or an anatomical disorder of the intestinal mucosa such as villous atrophy.

The form for administration of the BTL inhibitor of the present invention may include liquid, powders, granulations and tablets, but not limited thereto. Particularly when subjects for administration are humans, tablets or granulations that can be readily administered are preferable. The route of administration may be oral administration, but not limited thereto. In the case of administration to livestock animals, the present BTL inhibitor may preferably be in the form of powders and mixed with feed for oral administration, for ready handling.

Dosages of the BTL inhibitor of the present invention may preferably be 10⁶-10¹¹ cells/day, particularly 10⁸-10¹⁰ cells/day in terms of live Bacillus subtilis cells. When the subject is an animal raised for meat, it is preferable to administer the BTL inhibitor by adding the BTL inhibitor in the form of powders to the feed at a rate of 10³-10⁹ cells/g and particularly 10⁴-10⁷ cells/g in terms of live bacterial cells. Sufficient action may be obtained via dosages of 10⁶ cells/day or more. Efficient BTL inhibition may be obtained via dosages of 10¹¹ cells/day or less.

The BTL inhibitor of the present invention may enhance the intestinal barrier function of the subjects and may inhibit invasion of intestinal lymph nodes by harmful bacteria in the intestinal tract. As a result, what is known as opportunistic infection, multiple organ failure, and septicemia may be prevented. Administration of the present BTL inhibitor to humans may contribute to human health. Administration to animals such as those raised for meat can provide safe meat by preventing contamination thereof.

The present invention will be explained in further detail with reference to the Examples and Comparative Examples. However, the present invention is not limited thereto.

### Example 1

Eighteen six-week-old mice (strain: ICR, male) having uniform intestinal flora via 1 week of preliminary breeding after five weeks of age were divided into two groups of mice, that are the control group (10 mice) and the intake group (8 mice) . The control group was given feed "CE-2" manufactured by Clea Japan, Inc. The intake group was given feed "CE-2" containing 3 x 10⁷ CFU/g of *Bacillus subtilis* C-3102 (deposited at National Institute of Bioscience and Human Technology with deposition number FERM BP-1096). From the 14th day of the feeding, antibiotics was administered to the mice of both groups by feeding them with a water containing 0.8mg/ml penicillin and 2mg/ml streptomycin as a drinking water. Three days after the start of antibiotic administration, a microscopic examination of feces was performed. After confirmation that intestinal bacteria had been eliminated, 10⁸ CFU/animal of streptomycin-resistant *Escherichia coli (E.coli* C25; purchased from American Type Culture Collection; deposition number PTA-1896; deposition date May 19, 2000 and now publicly available) were administered. Afterwards, a water containing only streptomycin was given as a drinking water to the mice. Four days after administration of streptomycin-resistant *Escherichia* coli, mice were sacrificed and dissected in accordance with the following operation, for counting the number of bacteria in MLN and caecum.

Although not much difference was observed as to number of bacteria in caecum, number of bacteria in MLN of the intake group was 49% of that of the control group, i.e. notably lowered.

**Table 1**

| | Number of bacteria in MLN (CFU/MLN) | Number of bacteria in caecum (Log CFU/g) |
|---|---|---|
| Control group (n=10) | 105.3±77.3 | 9.28±0.19 |
| Intake group (n=8) | 51.9±31.5 | 9.45±0.15 |

### (Method for counting number of bacteria)

A mouse was sacrificed by dislocating vertebrae cervicales and sprayed with 70% alcohol. Abdominal integument was dissected and peritoneum was again disinfected with 70% alcohol. Peritoneum was then dissected and MLN and caecum were taken out. In this procedure, the operation apparatus used were disinfected by dipping in 100% alcohol and then burn to sterilize every time before changing the dissection part.

The MLN taken was homogenized in 1ml of BHI liquid medium. The homogenate was plated onto three DHL mediums containing streptomycin. The amount of the homogenate plated was 0.2ml per each medium. The homogenate on the medium was then cultured at 37°C for 20 hours. Number of bacteria per one MLN was determined from the number of colonies emerged.

The caecum taken was cut with scissors and placed in a 50 ml tube containing glass beads and 10ml of BHI liquid medium. The caecum including its content was weighed, and then vigorously stirred to suspend the content. The suspension was diluted 100 times with PBS (-) (trade name; manufactured by ROMAN KOGYO K.K. 9.57mM). This dilution was repeated three times so that the final dilution was 1 million times. The final diluted liquid was plated onto three DHL mediums containing streptomycin. The amount of the liquid plated was 0.1 ml per medium. The media were then cultured at 37°C for 20 hours. Number of bacteria per unit weight of caecum content was calculated from the number of colonies emerged.

### Example 2

Thirty six-week-old mice (strain: ICR, male) having uniform intestinal flora via 1 week of preliminary breeding after five weeks of age were divided into two groups of mice, that are the control group (15 mice) and the intake group (15 mice) . The control group was given feed "CE-2" manufactured by Clea Japan, Inc. The intake group was given feed "CE-2" containing 3 x 10⁷ CFU/g of *Bacillus subtilis* C-3102 (deposited at National Institute of Bioscience and Human Technology with deposition number FERM BP-1096). From the 14th day of the feeding, antibiotics was administered to the mice of both groups by feeding them with a water containing 0.8mg/ml penicillin and 2mg/ml streptomycin as a drinking water. Three days after the start of antibiotic administration, a microscopic examination of feces was performed. After confirmation that intestinal bacteria had been eliminated, 10⁸ CFU/animal of the same streptomycin-resistant *Escherichia* coli as used in Example 1 were administered. Afterwards, a water containing only streptomycin was given as a drinking water to the mice. Four days after administration of streptomycin-resistant *Escherichia coli*, mice were sacrificed and dissected by the same operation as in Example 1, for counting the number of bacteria in MLN and caecum.

Although not much difference was observed as to number of bacteria in caecum, number of bacteria in MLN of the intake group was 62% of that of the control group, i.e. notably lowered. The difference was statistically significant with the level of significance 5%.

**Table 2**

| | Number of bacteria in MLN (CFU/MLN) | Number of bacteria in caecum (Log CFU/g) |
|---|---|---|
| Control group (n=15) | 112.6±64.8 | 10.1±0.21 |
| Intake group (n=15) | 69.4±42.2* | 9.8±0.24 |

| | | |
|---|---|---|
| *P<0.05 | | |

### Example 3

Thirty-nine six-week-oldmice (strain: ICR, male) having uniform intestinal flora via 1 week of preliminary breeding after five weeks of age were divided into four groups of a BTL control group (18 mice) , a BTL intake group (17 mice) , a healthy control group (2 mice), and a healthy intake group (2 mice). The BTL control group and healthy control group were given feed "CE-2" manufactured by Clea Japan, Inc. The BTL intake group and healthy intake group were given feed "CE-2" containing 3 x 10⁷CFU/g of *Bacillus subtilis* C-3102 (deposited at National Institute of Bioscience and Human Technology with deposition number FERM BP-1096) . From the 14th day of administration, antibiotics was administered to the mice of the BTL control group and BTL intake group by feeding them with a water containing 0.8 mg/ml penicillin and 2.0 mg/ml streptomycin as a drinking water. Three days after the start of antibiotic administration, a microscopic examination of feces was performed. After confirmation that intestinal bacteria had been eliminated, 0.63 X 10⁸ CFU/animal of the same streptomycin-resistant *Escherichia* *coli* as used in Example 1 were administered. Afterwards, a water containing only streptomycin was given as a drinking water to the mice. Four days after administration of streptomycin-resistant *Escherichia coli*, mice were sacrificed and dissected. Mice in the healthy control group and healthy intake group to which antibiotics and streptomycin-resistant *Escherichia coli* were not administered were sacrificed and dissected on the same day as those in the BTL control group and BTL intake group. The ileum and caecum were taken out and fixed in a buffering solution (pH 7.2) containing 10% formalin by volume. Afterwards, samples were embedded in paraffin in the usual manner and slices with a thickness of 5 µm were prepared. Hematoxylin-eosin staining and PAS staining were then performed, and samples were observed under a light microscope.

The necrosis and detachment/desquamation of mucosal epithelial cells in the ileum and caecum as well as cellular infiltration in the lamina propria in each group were scored 0 for none, 1 for slight, 2 for moderate, and 3 for extensive and then the average was determined. Furthermore, the deviation in the BTL intake group and BTL control group was determined. Results are shown in Table 3.

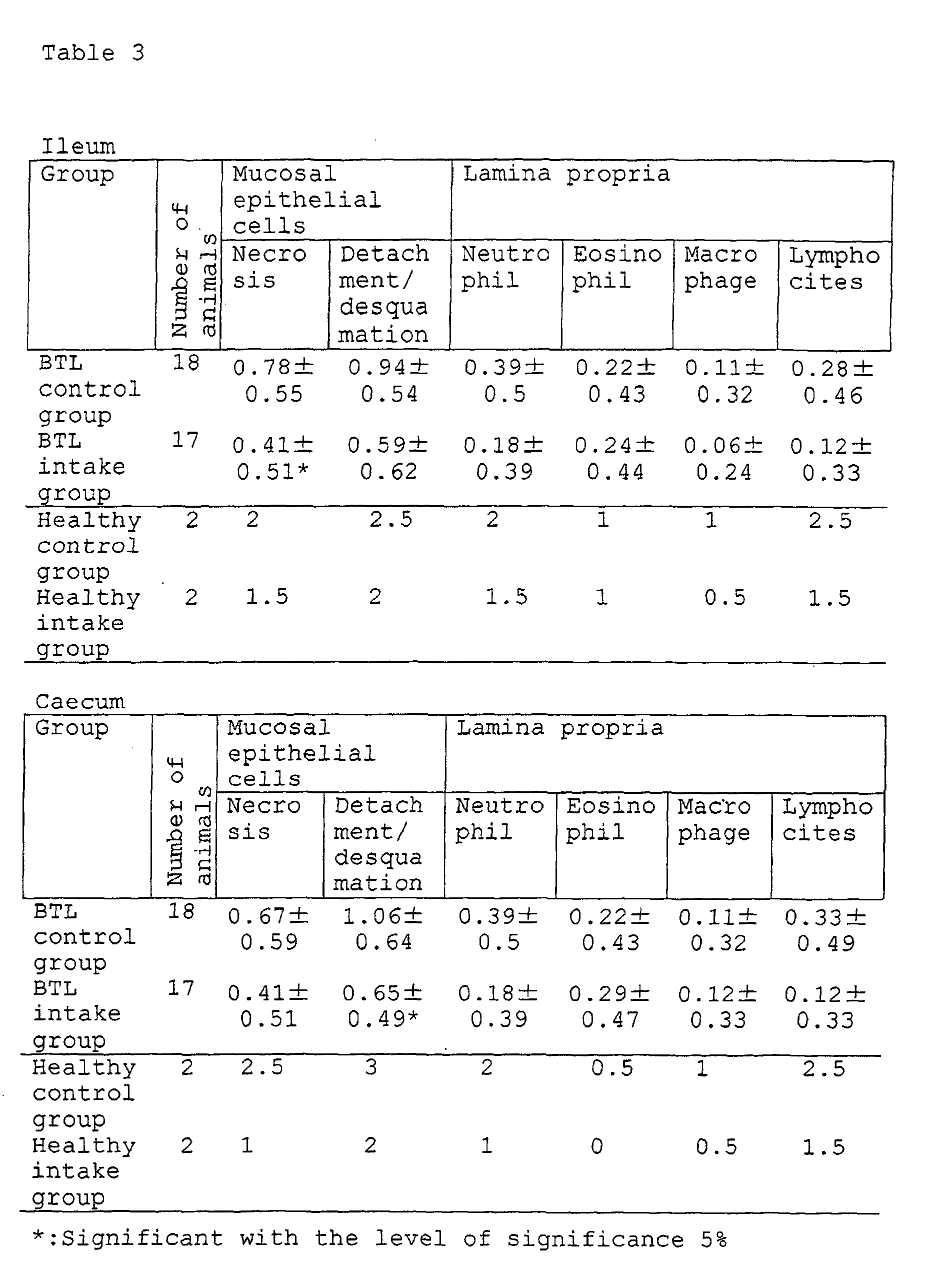

BTL was strongly elicited in both of the BTL intake group and BTL control group. The present BTL inhibitor was administered to the BTL intake group whereas not administered to the BTL control group. Comparing these groups, necrosis and detachment/desquamation of mucosal epithelial cells and cellular infiltration in the lamina propria in the BTL intake group were fewer than those in the BTL control group. Particularly, the difference was significant with the level of significance 5% as to necrosis in ileum and detachment/desquamation in caecum. Necrosis and detachment/desquamation of mucosal epithelial cells and cellular infiltration in the lamina propria of the healthy intake group were also fewer than those in the healthy control groups.

Necrosis and detachment/desquamation of mucosal epithelial cells and infiltration of neutrophiles and lymphocites in the lamina propria in the healthy intake group and healthy control group were more frequent than those in BTL intake group and BTL control group. This appears to be because the observation was made when damages in intestinal tract that had been occurred in BTL control group and BTL intake group by the action of antibiotics were restored.

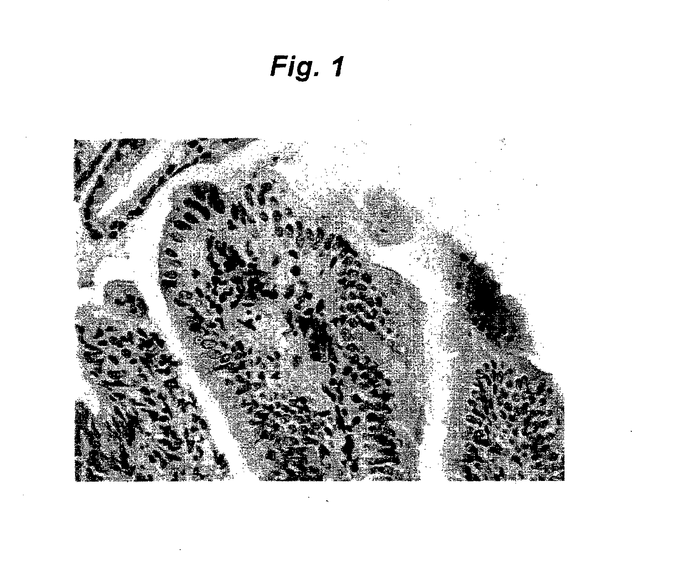

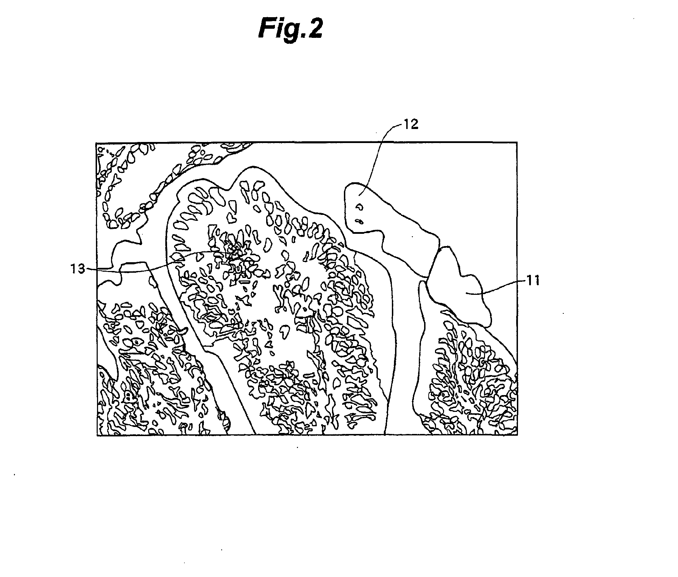

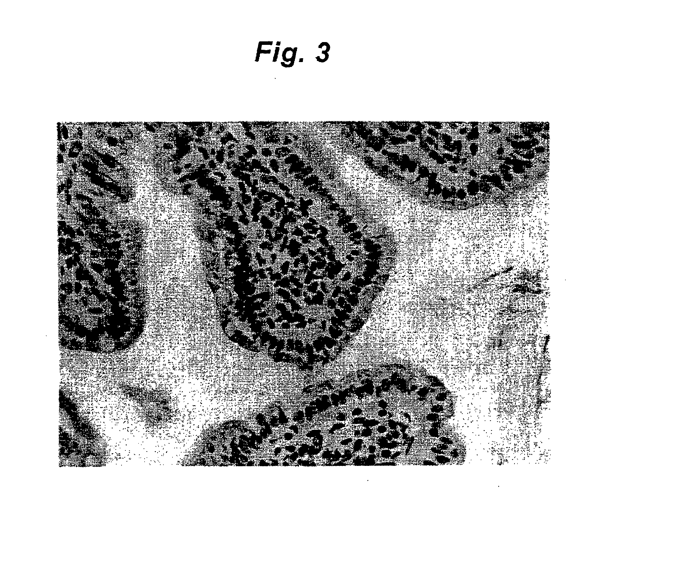

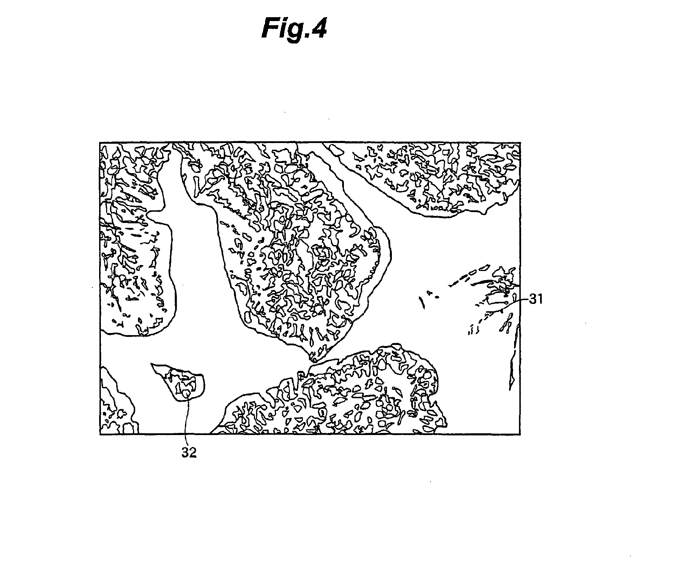

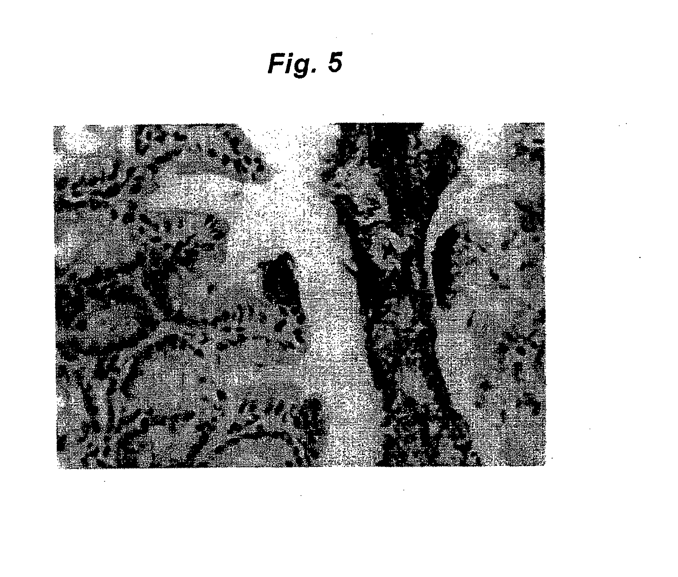

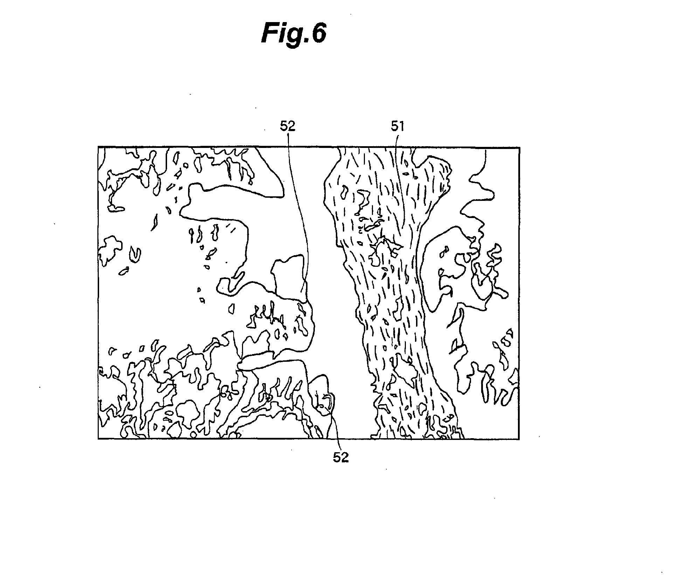

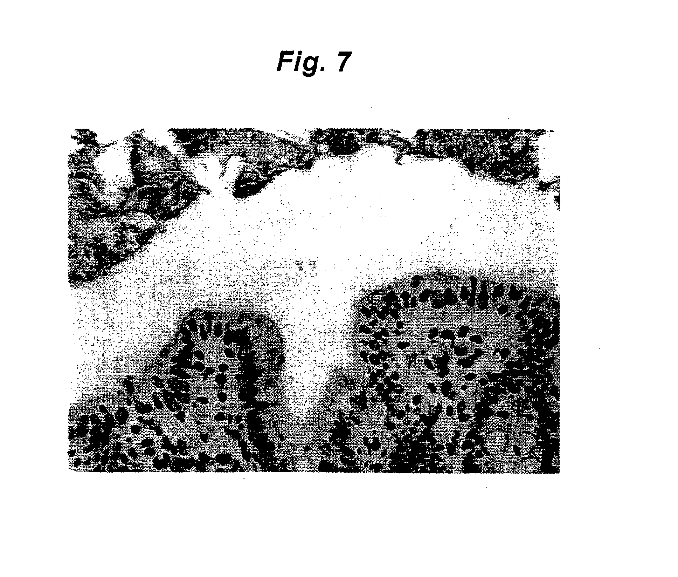

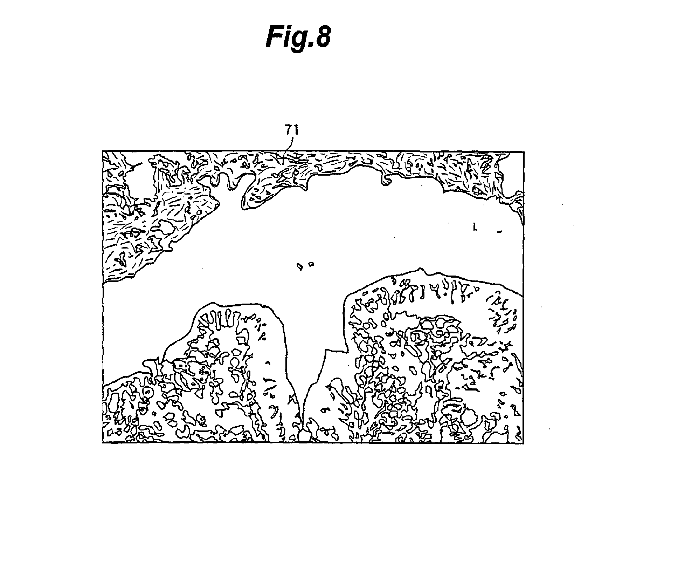

Microscopic photographs of ileum in the healthy control group and healthy intake group are shown in Figs. 1 and 3, respectively. Microscopic photographs of caecum in the healthy control group and healthy intake group are shown in Figs. 5 and 7, respectively. The location of necrosis and detachment/desquamation of mucosal epithelial cells and infiltration of neutrophiles and lymphocites in the lamina propria observed in Figs. 1, 3, 5 and 7 is illustrated in Figs. 2, 4, 6 and 8, respectively.

Numerals in Figs. 1 to 8 refer to the following:
11: part with nuclear disappearance, necrosis and desquamation, and dyed by eosinophilic staining
12: part with nuclear disappearance, necrosis and desquamation
13: part with infiltration by neutrophiles and lymphocites
31: bacteria
32: part with desquamation
51: bacteria
52: part with necrosis
71: bacteria

Necrosis and detachment/desquamation of mucosal epithelial cells were prominent in the ileum and caecum of the healthy control group. Further, neutrophil and lymphocyte infiltration into the lamina propria are frequently observed in the healthy control group (see Figs. 1 and 2 as well as 5 and 6). In contrast, the ileum and caecum of the healthy intake group displayed less necrosis and detachment/desquamation of mucosal epithelial cells, and neutrophil and lymphocyte infiltration of the lamina propria tended to be less frequent than those in the healthy control group. Outlines of a thick glycocalyx were clearly observed on the surface of mucosal epithelial cells (see Figs. 3 and 4 as well as 7 and 8) in the healthy intake group. From these results, it is apparent that administration of the present BTL inhibitor results in reduction of necrosis and detachment/desquamation of epithelial cells in the intestinal mucosa, reduction of invasion of intestinal tract tissue by bacteria, and reduction of neutrophil and lymphocyte infiltration into the lamina propria.

### Experiment 1

Eight strains, that are *Bacillus subtilis* C-3102 strain, *Bacillus amyloliquefaciens* ATCC 23350 strain (type strain),*Bacillus amyloliquefaciens* IFO 14141 strain, *Bacillus amyloliquefaciens* IFO 3022 strain, Bacillus subtilis IFO 3009 strain, *Bacillus subtilis* IFO 12112 strain, *Bacillus subtilis* ATCC 9372 strain and *Bacillus subtilis* ATCC 6051 strain (type strain) , were cultured overnight in 10 ml each of TS medium. The cells were then collected and washed.

*Bacillus amyloliquefaciens* ATCC 23350 strain, *Bacillus subtilis* ATCC 9372 strain and *Bacillus subtilis* ATCC 6051 strain are publicly available from American Type Culture Collection, and *Bacillus amyloliquefaciens* IFO 14141 strain, *Bacillus amyloliquefaciens* IFO 3022 strain, *Bacillus subtilis* IFO 3009 strain and *Bacillus subtilis* IFO 12112 strain are publicly available from Institute for Fermentation, Osaka, Japan.

Chromosomal DNA of each cultured strain was extracted and purified with the DNA preparation kit for yeast and Gram positive bacteria "GenTLE" (manufactured by TAKARA SHUZO CO., LTD.), to obtain chromosomal DNA solution. The extraction and purification was performed in accordance with the instructions of the DNA preparation kit except that vacuum-dried chromosomal DNA pellet was dissolved in 20 µl of TE buffer.

The resulting chromosomal DNA solution was admixed with other reagents as illustrated in Table 4, to prepare a sample for PCR treatment.

**Table 4**

| | |
|---|---|
| Chromosomal DNA solution | 1 µl |
| 10X PCR buffer | 5 µl |
| dNTP mixed solution | 8 µl (2.5mM each) |
| Primer 1 | 0.2 µM(Concentration in the final sample) |
| Primer 2 | 0.2 µM (Concentration in the final sample) |
| Taq Polymerase | 0.5 µl (5 units/µl) |
| Sterilized water | 34.5 µl |
| Total | 50 µl |

In Table 4, "10X PCR buffer" was the one attached to "TaKaRa LA-PCR kit Ver2" manufactured by TAKARA SHUZO CO., LTD. Primers 1 and 2 are those identified by Sequences 1 and 2, respectively. These are developed for amplifying a DNA fragment coding for alpha-amylase of Bacillus *amyloliquefaciens* having a length of about 2 kb, and having the sequences as follows:

Sequence 1:

5'-GCCCCGCACATACGAAAAGACTGGCTGAAA-3'

Sequence 2:

5'-GGATCCCACGTTGTGATTAAAAGCAGCGAT-3'

The sample for PCR treatment was then subjected to PCR treatment with "TaKaRa LA-PCR kit Ver2" and "TaKaRa PCT Thermal Cycler PERSONAL" manufactured by TAKARA SHUZO CO., LTD, to obtain a PCR amplified product. The thermal conditions for PCT treatment was as illustrated in Table 5.

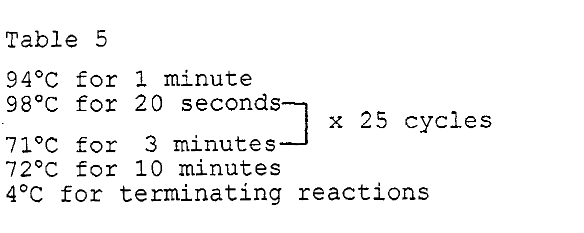

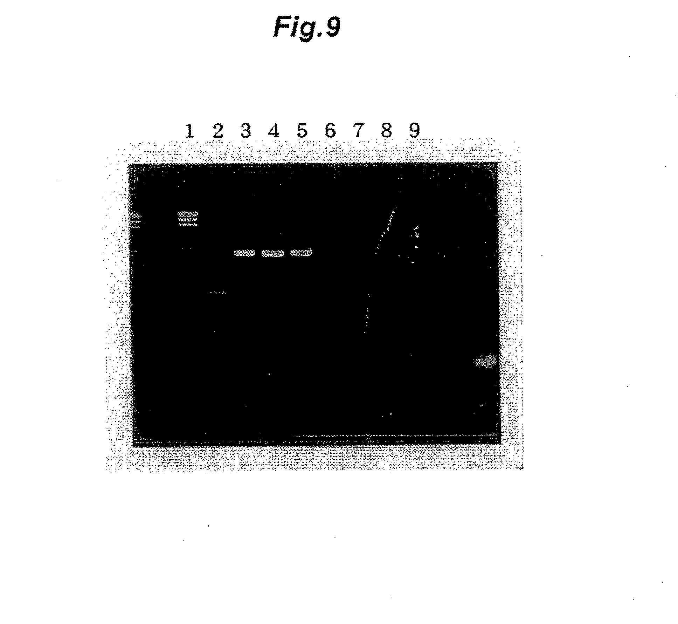

5 µl of the resulting PCR amplified product was taken and subjected to electrophoresis with 1.2% agarose gel in a horizontal electrophoresis unit Mupid 2 (manufactured by ADVANCE Co., Ltd.). As molecular weight markers, HindII digestion products of lambda-DNA were employed. The results of the electrophoresis are shown in Fig. 9.

In Fig. 9, numerals at the top of the photograph (1 to 9) are numbers of lanes. In each of the lanes, the following samples were subjected to electrophoresis:
Lane 1: Molecular weight marker (HindII digestion products of lambda-DNA) . There are recognized six bands in Lane 1. Three bright bands located at short migration distances are attributed to markers of 23130bp (the shortest migration distance), 9416bp (the second shortest migration distance) and 6557bp (the third shortest migration distance). The adjacent dark band located at longer migration distance is attributed to a marker of 4361bp. The two bands that are located at longer migration distances and slightly brighter than the dark band are attributed to markers of 2322bp (the second longest migration distance) and 2027bp (the longest migration distance).
Lane 2: Amplification product of DNA derived from *Bacillus subtilis* C-3102.
Lane 3: Amplification product of DNA derived from *Bacillus amyloliquefaciens* ATCC 23350 (type strain).
Lane 4: Amplification product of DNA derived from *Bacillus amyloliquefaciens* IFO 14141.
Lane 5: Amplification product of DNA derived from *Bacillus amyloliquefaciens* IFO 3022.
Lane 6: Amplification product of DNA derived from *Bacillus subtilis* IFO 3009.
Lane 7 : Amplification product of DNA derived from *Bacillus subtilis* IFO 12112
Lane 8: Amplification product of DNA derived from *Bacillus subtilis* ATCC 9372.
Lane 9: Amplification product of DNA derived from *Bacillus subtilis* ATCC 6051 (type strain).

In the electrophoresis results of the samples from *Bacillus amyloliquefaciens* strains (lanes 3, 4 and 5), an amplification product of a fragment having a length of about 2kb was observed. In the electrophoresis results of the samples from *Bacillus subtilis* IFO 3009 strain, *Bacillus subtilis* IFO 12112 strain, *Bacillus subtilis* ATCC 9372 strain and *Bacillus subtilis* ATCC 6051 strain ( lanes 6, 7, 8 and 9), no amplification product of a fragment was detected. However, in the electrophoresis result of the sample from *Bacillus subtilis* C-3102 (lane 2), a specific clear band of about 700kb was recognized.

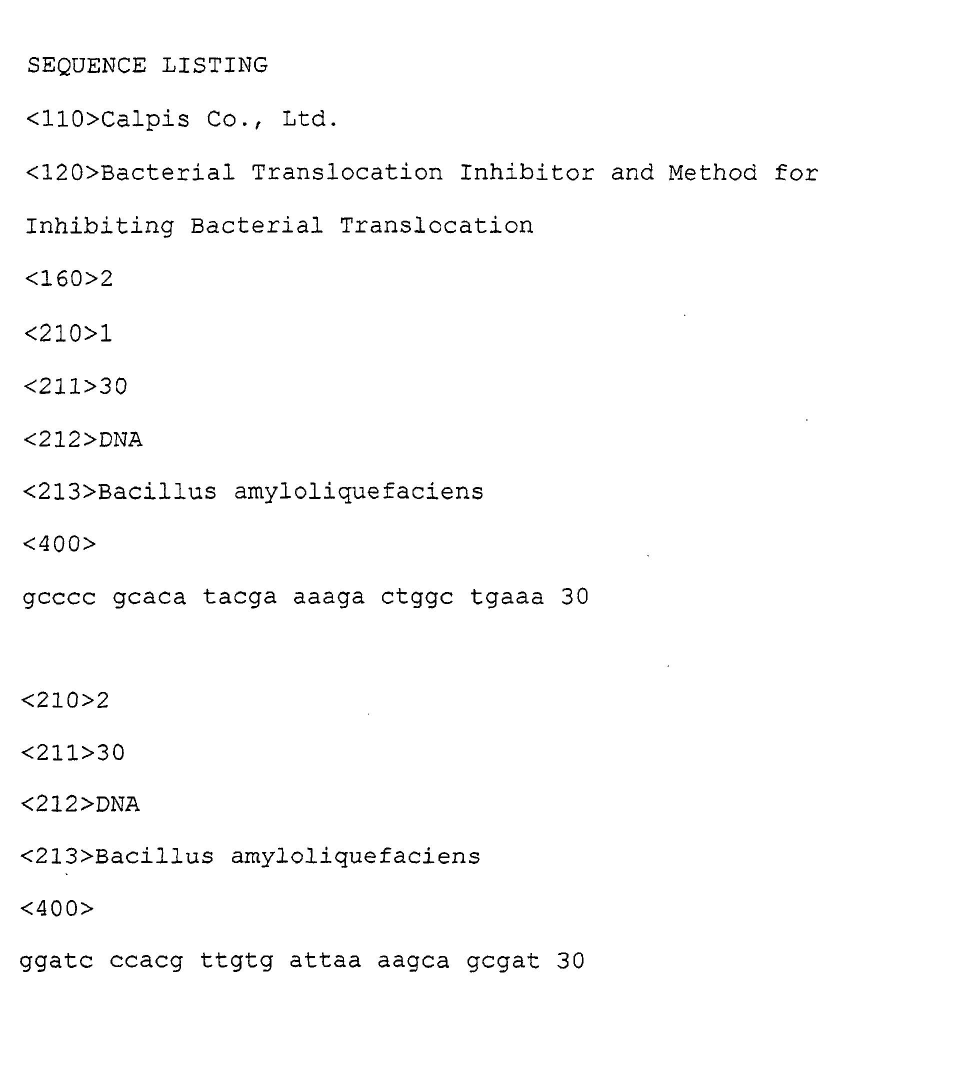

## Claims

1. A bacterial translocation inhibitor containing live *Bacillus subtilis* cells as an active ingredient.

2. The bacterial translocation inhibitor according to claim 1, wherein said *Bacillus subtilis* is *Bacillus subtilis* C-3102 (deposited at National Institute of Bioscience and Human-Technology with deposition number FERM BP-1096).

3. The bacterial translocation inhibitor according to claim 2, wherein said *Bacillus subtilis* C-3102 has a chromosomal DNA which gives a DNA fragment of about 700 bps when said chromosomal DNA is extracted, purified and subjected to PCR treatment using primers of sequences 1 and 2.

4. A method for inhibiting bacterial translocation comprising the step of orally administering to a subject in need thereof the effective amount of the bacterial translocation inhibitor of any one of claims 1-3.

5. The method for inhibiting bacterial translocation according to claim 4 wherein said subject is an animal other than human.

6. The method for inhibiting bacterial translocation according to claim 4 wherein said effective amount is in a range of 10⁶ to 10¹¹ cells/day in terms of live *Bacillus subtilis* bacteria cells.
